# EUROPEAN PATENT APPLICATION

(11) **EP 4 169 570 A1**
(43) Date of publication of application: **26.04.2023**
(21) Application number: 21203703.0
(22) Date of filing: 20.10.2021
(51) Int. Cl.: A61N 1/375, H01R 4/00, H01R 43/00

(54) **METHOD FOR PRODUCING AN ELECTRICAL FEEDTHROUGH**

(71) Applicant: BIOTRONIK SE & Co. KG, 12359 Berlin (DE)
(72) Inventor: WHITAKER, Colby, Oregon City, 97045 (US); PRIMAVERA, Anthony A., Newberg, 97132 (US)

(57) **Abstract**

A method for producing an electrical feedthrough (1) for an implantable medical device comprises: providing a plurality of conductors (2); arranging said plurality of conductors (2) in an electrically insulating material (7) such that the electrically insulating material (7) encloses said plurality of conductors (2) to form a subassembly (10) comprising said electrically insulating material (7) and said plurality of conductors (2); prior to, concurrently with or after said arranging, forming a bonding layer (8) of a biocompatible metal material on a circumferential surface (70) of said electrically insulating material (7); forming the electrical feedthrough (1) from the sub-assembly (10); and connecting the electrical feedthrough (1) to a flange element (9) by establishing a connection between the bonding layer (8) and the flange element (9).

## Description

The present invention relates to a method for producing an electrical feedthrough, to an electrical feedthrough as well as to an implantable medical device comprising such an electrical feedthrough.

All implantable medical devices that provide electrical stimulation or some sort of sensing functionality must have some form of electronics that monitor the patient, provide therapy, or both. The device electronics are generally not biocompatible and will cause adverse reactions with the patient if they were to come in direct or indirect contact with the patient. Hence, typically an implantable medical device comprises a titanium housing to separate the device electronics from the patient. The electronics must, however, have some direct electrical connection with the patient, e.g. in order to emit stimulation signals or to receive sense signals. The connection can be made using a hermetically sealed electrical feedthrough that allows for therapy to be applied to the patient from the device electronics and biological signals from the patient to be sensed by the device electronics. The electrical feedthrough component is complex and typically requires manual processes that significantly increase the cost of the feedthrough and can impact the quality of the feedthrough.

Known electrical feedthroughs often require individual interconnects to be brazed to a ceramic structure and typically involve a highly manual process and many components.

Furthermore, complex machined shapes are frequently required for supporting components (e.g. titanium flange). Also, electrical feedthroughs regularly comprise form factors that increase the circumferential path length of the electrical feedthrough (e.g. oblong/rectangular in shape) and reduce interconnect density.

Furthermore, solutions using multi-layered ceramic technology require successive layer build up to ensure hermetic properties.

Based on the above, it is an objective of the present invention to provide a method for producing an electrical feedthrough, an electrical feedthrough and an implantable medical device which allow for a simple, preferably semi-continuous manufacturing process, and enable high density interconnects.

In one aspect, a method for producing an electrical feedthrough for an implantable medical device comprises: providing a plurality of conductors; arranging said plurality of conductors in an electrically insulating material such that the electrically insulating material encloses said plurality of conductors to form a subassembly comprising said electrically insulating material and said plurality of conductors; prior to, concurrently with or after said arranging, forming a bonding layer of a biocompatible metal material on a circumferential surface of said electrically insulating material; forming the electrical feedthrough from the sub-assembly; and connecting the electrical feedthrough to a flange element by establishing a connection between the bonding layer and the flange element.

Accordingly, within the method a plurality of conductors is arranged within an electrically insulating material such that the electrically insulating material encloses the plurality of conductors. Prior to, concurrently with or after arranging the plurality of conductors in the electrically insulating material, a bonding layer is formed on a circumferential surface of the electrically insulating material, the bonding layer being made from a biocompatible material. In particular, the bonding layer may be formed on the electrically insulating material prior to arranging the conductors in the electrically insulating material. In another embodiment, the bonding layer may be formed on the electrically insulating material after the plurality of conductors already have been placed in the electrically insulating material.

From the assembly formed by the electrical conductors and the electrically insulating material the electrical feedthrough is formed. By means of the bonding layer, then, the electrical feedthrough is connected to a flange element.

The bonding layer is made from a biocompatible material. In one embodiment, the bonding layer may be formed from a palladium, platinum, platinum/iridium, niobium, or gold metal material.

The electrical feedthrough may particularly be formed to assume a disc shape. In particular, the electrical feedthrough may be formed from the subassembly comprising the electrically insulating material and the plurality of conductors by cutting the subassembly into a multiplicity of disc-shaped electrical feedthroughs.

In one embodiment, the disc-shaped electrical feedthrough is flat along a plane perpendicular to an axis, wherein the plurality of conductors extends through the electrically insulating material along the axis. In particular, the conductors may extend through the electrically insulating material such that a first face side of each conductor is exposed on a front side of the electrical feedthrough, and a second face side is exposed on a back side of the electrical feedthrough. Hence, an electrical connection to the electrical feedthrough may be established on the front side and the backside by connecting conductors, e.g. in the shape of conducting wires, to the electrical conductors extending through the electrical feedthrough.

In one embodiment, said forming the electrical feedthrough from the subassembly includes compressing the subassembly, and separating the subassembly into a plurality of disc-shaped electrical feedthroughs, particularly by cutting the (compressed) subassembly. The compressing serves to remove voids within the electrically insulating material, for example in between the electrically insulating material and the conductors.

The (compressed) subassembly may in particular be separated into a plurality of electrical feedthroughs each having a disc shape such that each electrical feedthrough comprises a plurality of conductors electrically insulated from one another by the insulating material and extending from a front side to a back side of the respective electrical feedthrough, wherein the electrically insulating material encloses the respective conductor laterally in a hermetically sealed fashion.

Preferably, in an embodiment, the arranging of the electrical conductors within the electrically insulating material is performed such that the subassembly and the respective electrical feedthrough comprise a cylindrical shape, respectively.

Particularly, compressing the subassembly is performed with an appropriate pressure that particularly may be chosen with regards to the properties of the layer of an electrically insulating material such as constituents (e.g., green sheet components, binder, solvent), moisture and the like. Appropriate pressures may lie in the range of 0.25 MPa to 0.75 MPa.

In one embodiment, the subassembly is heated e.g. after compressing the subassembly, particularly so as to sinter the electrically insulating material, for example a ceramic material, for example being made from a ceramic tape or alumina green tape. Due to the heating, a hermetic sealing of the feedthrough can be achieved. In some embodiments, the subassembly is heated to a temperature in the range of 600°C to 800°C, particularly in case of a ceramic material, such as a ceramic tape, comprising a LTCC (low-temperature-co-fired-ceramic). In some embodiments, the subassembly is heated to a temperature of at least 1200°C, particularly in case of a ceramic material, such as a ceramic tape, comprising a HTCC (high-temperature-co-fired-ceramic). In some embodiments, the subassembly is heated to a temperature of approx. 1000°C, in case of an electrically insulating material in the shape of an alumina green tape.

The aforementioned heating may be conducted before or after separating the subassembly into several electrical feedthroughs. Preferably, the heating is conducted before said separating.

In one embodiment, the step of connecting the electrical feedthrough to the flange element includes the forming of a weld connection in between the bonding layer and the flange element. For example, in one embodiment the feedthrough is placed in an opening of the flange element, and a hermetically sealed connection between the bonding layer and a circumferential (inner) face of the flange element surrounding the opening is established. Herein, the feedthrough may be placed within the opening for example at a controlled gap between the flange element and the feedthrough. For forming the weld connection for example a magnetic pulse welding process may be employed, the weld connection being formed by a weld seam extending circumferentially about the feedthrough.

In one embodiment, the electrically insulating material is one of or comprises one of:
- aluminium oxide (A1203),
- zirconium dioxide (ZrO2),
- a glass,
- a glass composed of 45 wt% SiO2, 24.5 wt% CaO, 24.5 wt% Na2O, and 6.0 wt% P2O5 (e.g. Bioglass),
- a glass comprising lanthanum,
- a ceramics,
- a ceramics containing apatite and wollastonite as predominant crystalline phases, (e.g. an apatite-wollastonite (AW) glass-ceramics),
- silicon nitride (Si3N4),
- silicon carbide (SiC), and/or
- aluminium nitride (AlN).

In one embodiment, the electrically insulating material may be provided in the shape of a layer, such as a ceramic tape, particularly an alumina green tape.

In one embodiment, the providing of a plurality of conductors includes arranging a plurality of conductors on an outer surface of a (preferably cylindrical) rod such that each conductor extends along a longitudinal axis of the rod, particularly parallel to one another. The conductors may be fixed to the rod in a suitable manner, e.g. by pressing them into the rod or pressing or drawing them through a capillary. Alternatively, the conductors may be made and thereby arranged by a photolithographic process including depositing a metal layer and etching away portions of the layer so as to form the individual conductors. According to another embodiment, the conductors may printed, e.g. onto the rod, and thereby arranged.

In one embodiment, the rod is formed from or comprises an electrically insulating material, for example the same electrically insulating material that is used for enclosing the electrical conductors. Particularly, the rod can be formed from a ceramics or alumina material or another suitable electrically insulating material.

In one embodiment, for arranging the plurality of conductors in the electrically insulating material, a layer of the electrically insulating material is wound such that the layer encloses the plurality of conductors to form the subassembly comprising the layer and the plurality of conductors. The layer may for example be made from a ceramics tape, such as an alumina green tape.

In one embodiment, subsequent to winding the (first) layer to enclose a (first) arrangement of electrical conductors, a further plurality of conductors is arranged on an outer surface of said wound layer, and a further layer of said electrically insulating material is wound around said further plurality of conductors. After winding the further layer, the subassembly formed in this way may be compressed so as to remove voids between the further layer and the conductors of said further plurality of conductors, and also in between the (first, inner) layer and the (first, inner) arrangement of electrical conductors in order to remove voids between this layer and the electrical conductors, if a compressing has not been performed before.

In one embodiment, said further layer is a ceramics tape, particularly an alumina green tape.

Particularly, in case a single layer of the electrically insulating material is wound onto a rod, the method can comprise a step in which, after compressing the subassembly, the subassembly is heated, particularly, so as to sinter the ceramics tape or alumina green tape when used as electrically insulating layer.

In an embodiment in which a further layer of an electrically insulating material is wound on a first, inner layer, after compressing the further layer and said further plurality of conductors enclosed laterally by the further layer and prior to separating the electrical feedthroughs, the entirety of layers and conductors can be heated for achieving an hermetic sealing of the final feedthroughs, particularly so as to sinter the ceramics tapes or alumina green tapes (or other materials that need heating/sintering, see above).

By providing a further arrangement of conductors and a further layer for enclosing the further arrangement of electrical conductors, electrical feedthroughs may be formed having an increased number of electrical conductors. A multi-layered subassembly herein may be fired to e.g. sinter the alumina green tape (or alternative material stated herein) and cut into discs of sufficient thickness to create hermetic electrical feedthroughs.

In one embodiment, the step of providing said plurality of conductors may include arranging a plurality of conductors on a surface of a spread (e.g. flat) layer of an electrically insulating material. Particularly, the conductors are arranged on this layer such that they extend parallel to one another. The conductors can be pressed into the layer or fixed to the layer to simplify the winding process.

In one embodiment, winding a layer of an electrically insulating material may include winding the layer in a spiral fashion, e.g. with conductors arranged thereon such that the layer encloses said plurality of conductors laterally to form a subassembly comprising said (spirally wound) layer and said plurality of conductors therein. Particularly, the wound layer may comprise several windings forming a spiral and enclosing said plurality of conductors laterally by way of said winding.

According to one embodiment, after compressing the (spirally wound) subassembly (particularly to ensure that no voids are present between the conductors and the wound layer), the subassembly may be heated for achieving a hermetic sealing of the final electrical feedthroughs. Particularly, said heating causes a sintering of the ceramics tape or alumina green tape (or of an alternative electrically insulating material stated herein).

In one embodiment, the conductors of the respective electrical feedthrough are formed out of or comprise one of the following materials: palladium (Pd), platinum (Pt) platinum/iridium, niobium, gold, nickel or copper.

In one embodiment, each conductor of an electrical feedthrough comprises an exposed face side on a front side of the feedthrough and an exposed face side on a back side of the feedthrough. The face sides on the front side are preferably flush with the front side and the faces sides on the back side are preferably flush with the back side of the electrical feedthrough (e.g. by way of separating the individual electrical feedthrough from the subassembly).

According to one embodiment, a tab of a solderable material is laser welded to the face side of each conductor on the back side of an electrical feedthrough separated from the subassembly.

According to a further embodiment, an electronic module of an implantable medical device (e.g. a cardiac pacemaker), particularly a printed circuit board assembly (PCBA), is soldered to the tabs. Particularly, the respective tab can be formed out of copper (Cu), wherein the respective tab can comprise a surface plating such as ENIG (for Electroless Nickel Immersion Gold). Such an ENIG plating consists of an electroless nickel plating covered with a layer of immersion gold.

In one embodiment, a header wiring element of a header of the implantable medical device is laser welded to the face side of each conductor on the front side of the feedthrough. In particular, the header may be configured to connect at least one component (e.g. an electrode lead) to the implantable medical device (e.g. cardiac pacemaker), so as to connect the component to an electronic module of the implantable medical device that is enclosed by the housing.

In one embodiment, the insulating material directly contacts each of the plurality of conductors, i.e. without a solder or the like between the conductor and the insulating material.

In another aspect, an electrical feedthrough is produced using the method as described above.

In yet another aspect, an assembly of an electrical feedthrough for an implantable medical device comprises a plurality of conductors, wherein each conductor comprises an exposed face side on a front side of the electrical feedthrough and an exposed face side on a back side of the electrical feedthrough; an electrically insulating material enclosing said plurality of conductors and forming a circumferential surface; a bonding layer of a biocompatible metal material placed on said circumferential surface of said electrically insulating material; and a flange element hermetically connected to the bonding layer and configured to establish a hermetic connection to a housing portion of a housing of the implantable medical device.

According to a further aspect, an implantable medical device, particularly an implantable cardiac pacemaker, comprises an assembly comprising an electrical feedthrough of the kind described before.

According to an embodiment of the implantable medical device, a tab is connected, particularly laser welded, to the face side of each conductor on the back side of the feedthrough. Particularly, the respective tab can be formed out of copper (Cu), wherein the respective tab can comprise a surface plating such as ENIG (see also above).

According to a further embodiment of the implantable medical device, the implantable medical device comprises an electronic module (particularly a printed circuit board assembly (PCBA)) that is connected, particularly soldered, to the tabs.

According to a further embodiment of the implantable medical device, the implantable medical device comprises a housing for accommodating the electronic module.

Particularly, the housing comprises titanium or a titanium alloy or is formed out of titanium or a titanium alloy. Alternatively, the housing may comprise a biocompatible plastic such as LCP, silicone or epoxy or may be formed out of the biocompatible plastic.

According to a further embodiment of the implantable medical device, the electrical feedthrough is connected to the housing via the flange element. Particularly, the flange element is formed out of titanium or a titanium alloy or comprises titanium or a titanium alloy.

In one embodiment, the flange element is laser welded to the housing.

According to a further embodiment of the implantable medical device, the implantable medical device comprises a header configured to provide an electrical connection between a component of the implantable medical device (such as an electrode lead, e.g. in case the implantable medical device is an implantable cardiac pacemaker) and the electronic module or PCBA enclosed by the housing of the implantable medical device.

Further, according to an embodiment of the implantable medical device, the header comprises header wiring elements for electrically connecting the header to the conductors of the electrical feedthrough, wherein each header wiring element is laser welded to the face side of a conductor of the electrical feedthrough on the front side of the electrical feedthrough.

The various features and advantages of the present invention may be more readily understood with reference to the following detailed description and the embodiments shown in the drawings. Herein,
- Fig. 1: shows an embodiment of a method for producing an electrical feedthrough according to the present invention;
- Fig. 2A: shows a front side of an electrical feedthrough;
- Fig. 2B: shows a back side of the electrical feedthrough;
- Fig. 3: illustrates another embodiment of producing an electrical feedthrough by winding a layer; and
- Fig. 4: illustrates a wound layer for producing an electrical feedthrough;
- Fig. 5: shows a subassembly formed by electrical conductors enclosed in an electrically insulating material;
- Fig. 6: shows a cross-sectional view of a single conductor of an embodiment of an electrical feedthrough according to the present invention;
- Fig. 7: shows an embodiment of an electrical feedthrough, comprising a bonding layer of a biocompatible metal material on a circumferential outer surface of an electrically insulating material;
- Fig. 8: shows the electrical feedthrough of Fig. 7 prior to arranging the electrical feed-through in a flange element for connecting the feedthrough to a housing of an implantable medical device;
- Fig. 9: shows a view of the feedthrough arranged in the flange element;
- Fig. 10: shows a front view of the feedthrough arranged in the flange element;
- Fig. 11: shows a schematic view of an implantable medical device in a patient; and
- Fig. 12: shows a schematic view of an implantable medical device, comprising a header for connecting electrode leads to the implantable medical device, and electronics enclosed in a housing.

Subsequently, embodiments of the invention shall be described in detail with reference to the drawings. In the drawings, like reference numerals designate like structural elements.

It is to be noted that the embodiments are not limiting for the invention, but merely represent illustrative examples.

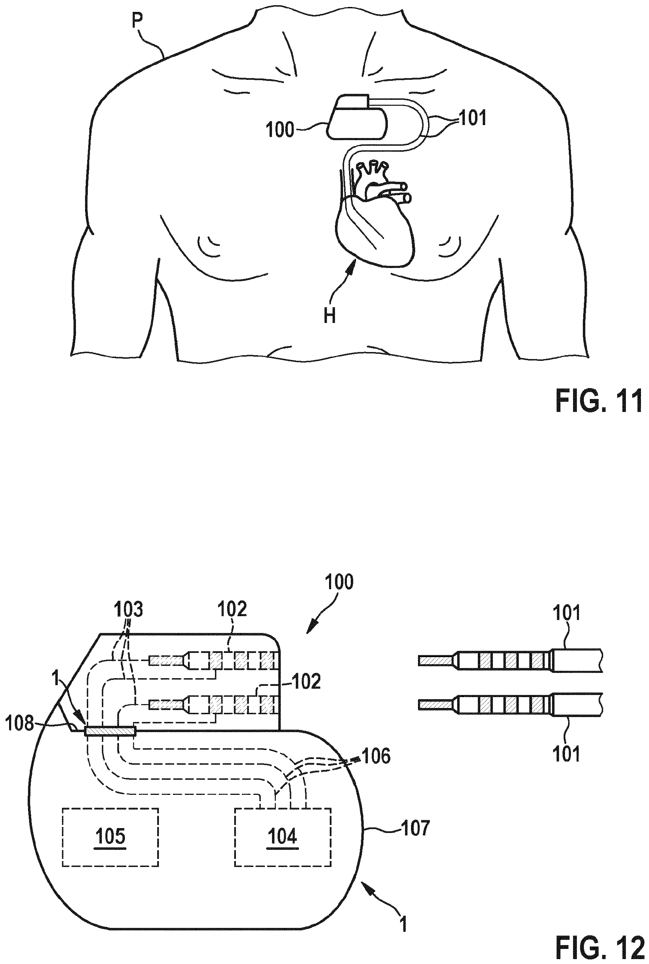

Referring first to Fig. 11, an implantable medical device 100, for example in the shape of a generator of a stimulation device, such as a pacemaker device, may for example be subcutaneously implanted in a patient P, wherein for example electrode leads 101 may extend from the implantable medical device 100 into a patient's heart to provide a therapeutic and/or diagnostic function on the patient P.

Referring now to Fig. 12, an implantable medical device 100 generally comprises a housing 107 which encloses functional components of the device, for example electronics 104 and an energy storage 105, particularly in the shape of a battery. The housing 107 herein serves to enclose the components arranged therein in a hermetic fashion, such that the components arranged in the housing 107 may not come into contact with tissue and bodily fluids in the surrounding of the implantable medical device 100.

The electrode leads 101 generally are connected to the implantable medical device 100, for example in the shape of a generator of a stimulation device, by means of plug connectors 102, into which connectors of the leads 101 may be inserted in order to establish an electrical connection in between the leads 101 and the implantable medical device 100. The plug connectors 102 herein are arranged on a header of the implantable medical device 100, wherein an electrical connection in between contacts of the plug connectors 102 is to be established to the device electronics 104.

For this, an electrical feedthrough 1 is provided at a housing portion 108 separating the header from the housing 107, the feedthrough 101 providing for an electrical interconnection of connecting wires 103 on the side of the header and connecting wires 106 on the side of the housing 107, such that the plug connectors 102 are by means of the feedthrough 1 electrically interconnected with the electronics 104 encapsulated within the housing 107.

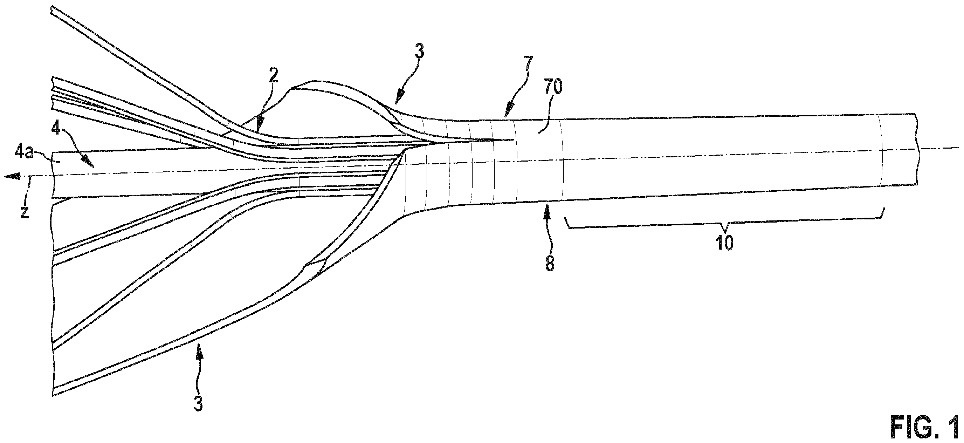

Fig. 1 illustrates an embodiment of a method for manufacturing an electrical feedthrough 1 for an implantable medical device 100.

As shown in Fig. 1, a plurality of conductors (e.g. in form of palladium or platinum wires) 2 is provided, particularly by arranging the individual conductor 2 on an outer surface 4a of a rod 4 such that the conductors extend along a longitudinal axis z of the rod 4 as well as parallel to one another (see Fig. 1 on the left).

Subsequently, a layer 3 comprising an electrically insulating material 7, such as alumina, is placed on the conductors 2 so that the latter are covered along the circumference of the rod 4 (see Fig. 1 in the middle). The layer 3 can be an alumina green tape. The rod 4 can also be formed out of alumina. Other electrically insulating materials can also be used for the layer 3 and rod 4.

By winding the layer 3 on the conductors 2, the conductors 2 are laterally enclosed by the layer 3. In order to eliminate voids between the conductors 2 and the layer 3 and the rod 4, the subassembly 10 consisting of the conductors 2, the wound layer 3 and the rod 4 is compressed (see Fig. 1 on the right).

The compressed rod 4, the conductors 2, and the layer 3 can then have additional layers of conductors (e.g. palladium or platinum wires) 2 and alumina green sheet 3 added to the circumference to achieve the sufficient quantities of interconnects. The cylindrical assembly is then fired to sinter the alumina green tape and cut into discs of sufficient thickness to produce hermetic electrical feedthroughs 1.

The method as illustrated in Fig. 1 allows manufacturing hermetic electrical feedthroughs 1 in a semi-continuous process flow using e.g. ceramic green (e.g. alumina) tape and conductors (e.g. metal traces or wire comprising palladium or platinum) in/on the green tape.

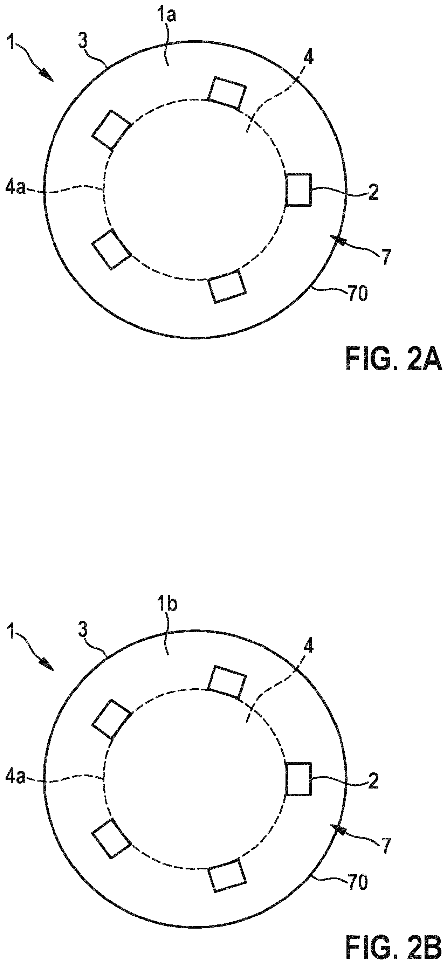

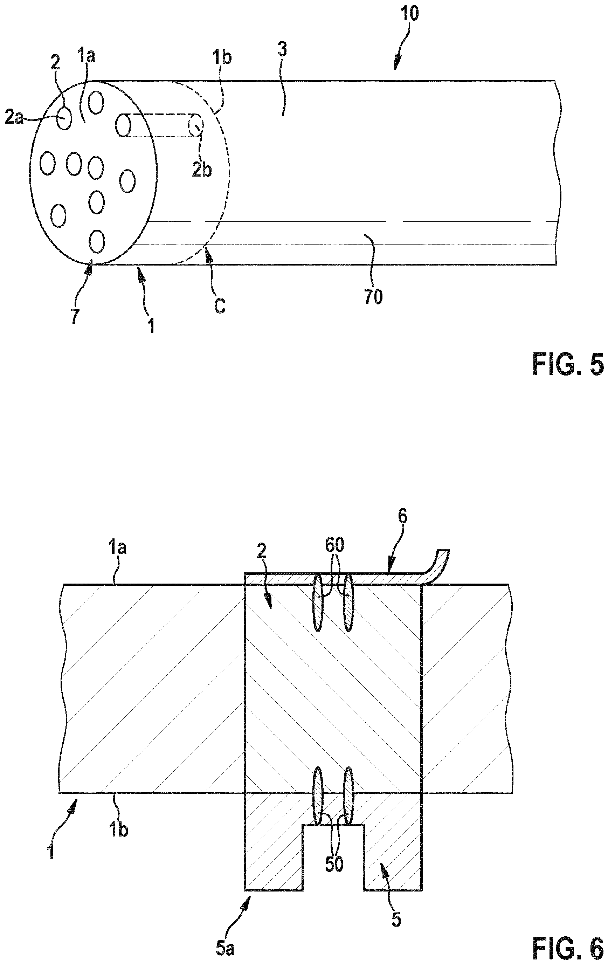

An electrical feedthrough 1 produced in this way comprises, as illustrated in Figs. 2A and 2B, a front side 1a having exposed face sides 2a of the conductors 2 (see Fig. 2A) as well as a back side 1b having exposed face sides 2b of the conductors 2 (see Fig. 2B). The face sides 2a, 2b of the conductors 2 can be electrically contacted, as will be described in more detail below in conjunction with Fig. 6.

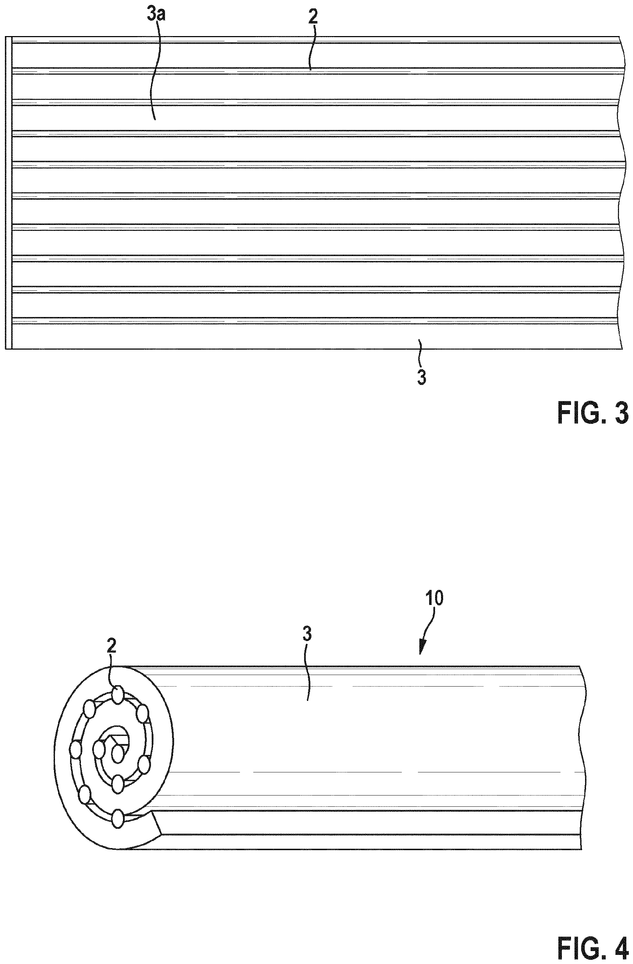

Figs. 3 to 5 illustrate a further embodiment of a method for manufacturing a feedthrough 1.

In this embodiment, the electrical feedthroughs 1 are produced by spirally rolling a layer 3 of an electrically insulating material 7 (e.g. an alumina green tape). As shown in Fig. 3 conductors 2 (e.g. palladium or platinum wires) are arranged on a surface 3a of the spread layer 3. Particularly, the conductors 2 (Pt, Pd, etc.) may be pressed into and distributed along the length of the layer 3, wherein a spacing between the conductors 2 can be constant (as shown in Fig, 3), or may be variable depending e.g. on design optimization.

Once the conductors 2 are arranged on the spread-out layer 3, the layer 3 (e.g. ceramic or alumina green tape) and conductors 2 thereon can be cut to a desired size if necessary. Thereafter, the layer 3 is rolled to form a spiral thereby enclosing the conductors 2 as shown in Fig. 4.

A subassembly 10 formed by means of the method as illustrated in Fig. 1 or by means of the method as illustrated in Figs. 3 to 5 may be compressed to remove voids and heated to sinter the electrically insulating material to yield a hermetic structure. The compressed and heated subassembly 10 can then be separated into individual electrical feedthroughs 1 (e.g. by cutting along the dashed line C as shown in Fig. 5). The feedthroughs 1 each comprise a front side 1a and a back side 1b having exposed face sides 2a, 2b (cf. also Fig. 2A and 2B) of the conductors 2 that can be electrically contacted.

While soldering connections may be established directly on the face sides 2a, 2b of the conductors 2, due to a potential formation of brittle intermetallics of Pd and Sn it is not preferred to form a direct solder connection to the exposed surfaces of the hermetic feedthrough's conductors 2. To enable connection of the feedthrough 1 to an electronic module or printed circuit boards assembly (PCBA) in the medical device, one approach shown in Fig. 6 is to laser-weld a tab 5 of a solderable material (e.g. such as Cu with an ENIG surface finish) to the face side 2b of the respective conductor 2 on the back side 1b of the feedthrough 1, which tab 5 is beneficially of substantially the same size as the exposed face side 2b of the associated conductor 2. Fig. 6 illustrates a corresponding weld seam 50.

The respective tab 5 then may be used to form a solder joint with the electronic module/PCBA. Particularly, the process of attaching the respective tab 5 to the cylindrical disc feedthrough 1 can be automated with use of alignment fixtures and laser welding on an x-y stage with multiple feedthroughs 1 on the fixture.

A connection with an external header wiring element 6 may be formed by laser welding the wiring element 6 to the corresponding face side 2a of a conductor 2 on the front side 1a of the feedthrough 1, as shown in Fig. 6. Also shown is the weld seam 60 between the wiring element 6 and the conductor 2.

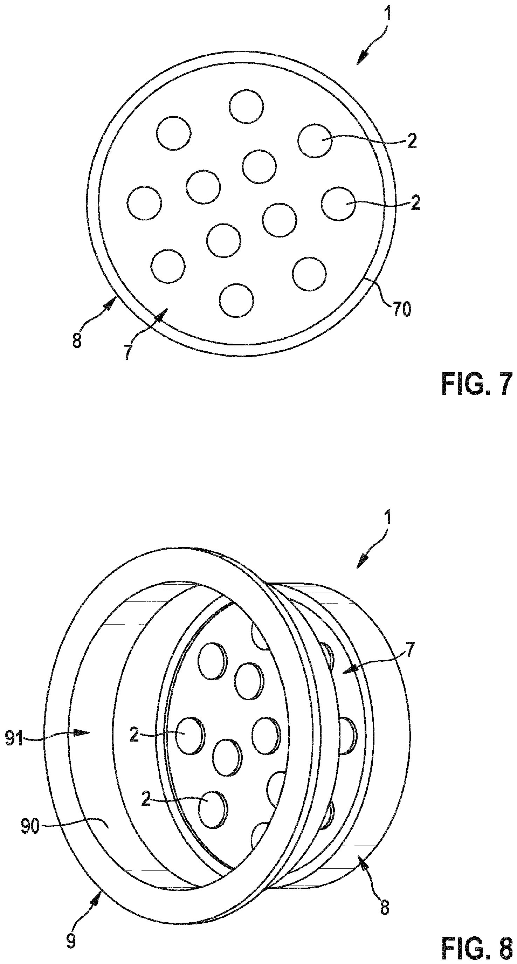

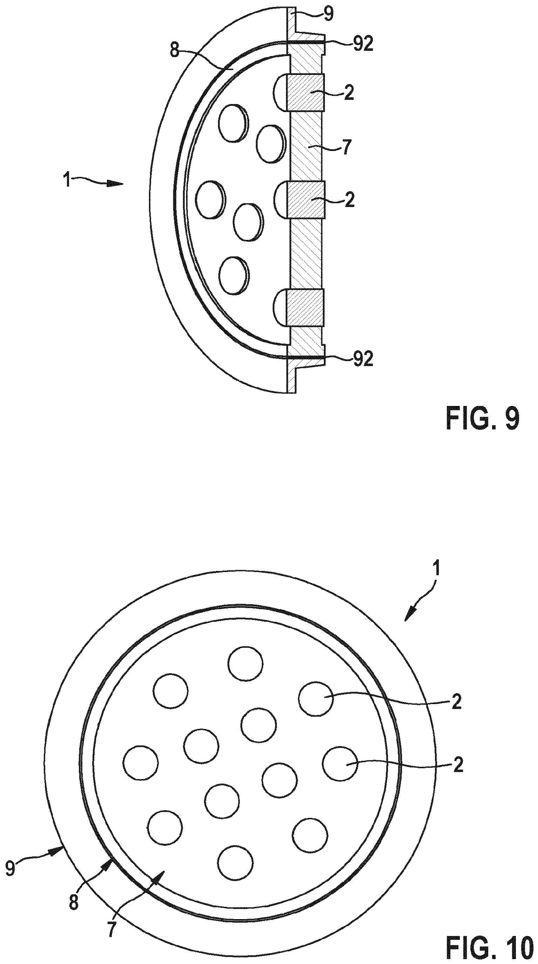

For connecting a feedthrough 1 to an associated housing portion 108 of a housing 107 of an implantable medical device 100, the feedthrough 1 is received in a flange element 9, as this is shown in Figs. 7 to 10. Herein, the flange element 9 may be formed from a titanium or titanium alloy material and allows for laser welding onto a titanium or titanium alloy housing 107 of the implantable medical device 100.

In order to provide a hermetic arrangement of the feedthrough 1 on the housing 107, the connection with the flange element 9 needs to be hermetic. Hence, for connecting the feedthrough 1 with the flange element 9, the feedthrough 1 is placed within an opening 91 of the flange element 9 circumferentially surrounded by a circumferential face 90, the flange element 9 in the shown embodiment comprising a ring shape for receiving the cylindrical feedthrough 1.

For establishing a connection in between the feedthrough 1 and the flange element 9, a bonding layer 8 is provided on an outer surface 70 of the electrically insulating material 7 enclosing the conductors 2. The bonding layer 8 is made from a biocompatible metal material, for example a palladium metal material, such that a contact of the bonding layer 8 with tissue or bodily fluids does not cause adverse effects to the patient.

Referring again to Fig. 1, the bonding layer 8 may be formed on an outer surface 70 of the layer 3 of the electrically insulating material 7 prior to enclosing the electrical conductors 2 using the layer 3. In another embodiment, the bonding layer 80 may be formed on the surface 70 of the electrically insulating material 7 after enclosing the conductors 2 with the layer 3, potentially after compressing and heating the layer 3.

Referring now again to Figs. 7 to 10, by means of the bonding layer 8 the feedthrough 1 is connected to the flange element 9 by forming a weld connection 92 in the shape of a weld seam within a gap in between the bonding layer 8 and the circumferential face 90 of the flange element 9. The weld seam surrounds the feedthrough 1, such that the gap in between the bonding layer 8 and the flange element 9 is closed and the feedthrough 1 is hermetically connected to the flange element 9.

The manufacturing process described herein advantageously allows use of continuous components for manufacture of electrical feedthroughs 1 by e.g. suitably rolling of e.g. ceramic green sheet 3 and metal traces 2 to yield a cylindrical shaped electrical feedthrough 1 with a simple manufacturing method.

By providing the bonding layer 8, no brazing is required for connecting the feedthrough 1 to a housing 107 of an implantable medical device 100. Rather, a weld connection may be formed in between the bonding layer 8 and the flange element 9 receiving the feedthrough 1, hence easing manufacturing and rendering the manufacturing process reliable, while allowing for a low-cost and high-volume procedure.

Electrical feedthroughs 1 produced as described herein may comprise a high interconnect density. Furthermore, a highly automatable semi-continuous process flow with reducing manufacturing complexity can be used. Furthermore, processing and costs of other components, such as the titanium flange, can be reduced.

It should be noted that the feedthrough 1 may comprise a shape different from a cylindrical shape, such as a substantially rectangular shape.

### LIST OF REFERENCE NUMERALS

- 1: Feedthrough
- 1a: Front side
- 1b: Back side
- 10: Subassembly
- 2: Conductors
- 2a, 2b: Face sides
- 3: Layer
- 3a: Surface
- 4: Rod
- 4a: Surface
- 5: Tab
- 50: Weld seam
- 6: Wiring element
- 60: Weld seam
- 7: Insulating material
- 70: Surface
- 8: Bonding layer
- 9: Weld flange
- 90: Circumferential face
- 91: Opening
- 100: Implantable medical device
- 101: Lead
- 102: Connector
- 103: Connecting wire
- 104: Device electronics
- 105: Energy storage
- 106: Connecting wire
- 107: Housing
- 108: Housing portion
- H: Heart
- P: Patient
- z: Axis

## Claims

1. A method for producing an electrical feedthrough (1) for an implantable medical device, comprising:
providing a plurality of conductors (2);
arranging said plurality of conductors (2) in an electrically insulating material (7) such that the electrically insulating material (7) encloses said plurality of conductors (2) to form a subassembly (10) comprising said electrically insulating material (7) and said plurality of conductors (2);
prior to, concurrently with or after said arranging, forming a bonding layer (8) of a biocompatible metal material on a circumferential surface (70) of said electrically insulating material (7);
forming the electrical feedthrough (1) from the sub-assembly (10); and
connecting the electrical feedthrough (1) to a flange element (9) by establishing a connection between the bonding layer (8) and the flange element (9).

2. The method according to claim 1, wherein said forming the electrical feedthrough (1) from the sub-assembly (10) includes: forming the feedthrough (1) to have a disc-shape.

3. The method according to claim 2, wherein said disc-shape is flat along a plane perpendicular to an axis (z), said plurality of conductors (2) extending through the electrically insulating material (7) along said axis (z).

4. The method according to one of claims 1 to 3, wherein said forming the electrical feedthrough (1) from the sub-assembly (10) includes:
compressing the subassembly (10), and
separating the subassembly (10) into a plurality of disc-shaped electrical feedthroughs (1).

5. The method according to claim 4, wherein after compressing the subassembly (10) the subassembly (10) is heated.

6. The method according to one of the preceding claims, wherein said connecting the electrical feedthrough (1) to the flange element (9) includes: forming a weld connection in between the bonding layer (8) and the flange element (9).

7. The method according to one of the preceding claims, wherein said connecting the electrical feedthrough (1) to the flange element (9) includes: placing the feedthrough (1) in an opening (91) of the flange element (9) and establishing a hermetically sealed connection between the bonding layer (8) and a circumferential face (90) of the flange element (9) extending about said opening (91).

8. The method according to one of the preceding claims, wherein the electrically insulating material (7) is one of or comprises at least one of: aluminium oxide, zirconium dioxide, a glass, a glass composed of 45 wt% SiO2, 24.5 wt% CaO, 24.5 wt% Na2O, and 6.0 wt% P2O5, a glass comprising lanthanum, a ceramics, a ceramics containing apatite and wollastonite as predominant crystalline phases, silicon nitride, and silicon carbide, aluminium nitride.

9. The method according to one of the preceding claims, wherein the providing the plurality of conductors (2) includes: arranging the plurality of conductors (2) on an outer surface (4a) of a rod (4) such that each conductor (2) extends along a longitudinal axis (z) of the rod (4).

10. The method according to one of the preceding claims, wherein said arranging said plurality of conductors (2) in said electrically insulating material (7) includes: winding a layer (3) comprising the electrically insulating material (7), such that the layer (3) encloses said plurality of conductors (2) to form said subassembly (10) comprising said layer (3) and said plurality of conductors (2).

11. The method according to claim 10, wherein said layer (3) is a ceramics tape, particularly an alumina green tape.

12. The method according to claims 10 or 11, wherein the method further comprises:
arranging a further plurality of conductors on an outer surface of said layer (3) and
winding a further layer comprising an electrically insulating material around said further plurality of conductors to form said subassembly, and compressing the subassembly.

13. The method according to one of claims 10 to 12, wherein said winding said layer (3) includes: winding the layer (3) in a spiral fashion with the conductors (2) arranged thereon such that the layer (3) encloses said plurality of conductors (2) to form said subassembly (10) comprising said layer (3) and said plurality of conductors (2).

14. An electrical feedthrough (1) produced using the method according to one of the preceding claims.

15. An assembly of an electrical feedthrough (1) for an implantable medical device, comprising:
a plurality of conductors (2), wherein each conductor (2) comprises an exposed face side (2a) on a front side (1a) of the electrical feedthrough (1) and an exposed face side (2b) on a back side (1b) of the electrical feedthrough (1);
an electrically insulating material (7) enclosing said plurality of conductors (2) and forming a circumferential surface (70);
a bonding layer (8) of a biocompatible metal material placed on said circumferential surface (70) of said electrically insulating material (7); and
a flange element (9) hermetically connected to the bonding layer (8) and configured to establish a hermetic connection to a housing portion (108) of a housing (107) of the implantable medical device (100).
